(19) Europäisches Patentamt
European Patent Office
Office européen des brevets

(11) Veröffentlichungsnummer: **0 227 598**
**B1**

(12) **EUROPÄISCHE PATENTSCHRIFT**

(45) Veröffentlichungstag der Patentschrift: **02.08.89**

(51) Int. Cl.⁴: **C 08 G 73/12**, C 07 D 207/448, B 32 B 27/04

(21) Anmeldenummer: **86810578.4**

(22) Anmeldetag: **10.12.86**

(54) **Stabile imidenthaltende Stoffgemische.**

(30) Priorität: **16.12.85 US 809444**

(43) Veröffentlichungstag der Anmeldung: **01.07.87 Patentblatt 87/27**

(45) Bekanntmachung des Hinweises auf die Patenterteilung: **02.08.89 Patentblatt 89/31**

(84) Benannte Vertragsstaaten: **CH DE FR GB IT LI NL**

(56) Entgegenhaltungen:
**EP-A-0 014 816**
**US-A-4 130 564**
**US-E-29 316**

(73) Patentinhaber: **CIBA- GEIGY AG, Klybeckstrasse 141, CH- 4002 Basel (CH)**

(72) Erfinder: **Chaudhari, Mohammad A., 47 Redwood Drive, Bethel Connecticut 06801 (US)**
Erfinder: **King, John J., 22 Evergreen Place, Ridgefield Connecticut 06877 (US)**

**EP 0 227 598 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Verwendung von Polymaleinimiden zur Herstellung verschiedener Polyadditions- und Polymerisationsprodukte ist bekannt. Besonders oft werden Bis-maleinimide verwendet, welche eine hohe thermische Stabilität aufweisen, und daher für die Herstellung von hochwertigen Verbundstoffen eingesetzt werden.

Die bekannten Bis-maleinimidsysteme enthalten aromatische eine und Alkenylphenole als Koreaktanten. Reaktionsprodukte ungesättigter Bisimide mit Aminen werden beispielsweise in US-3 658 764 und Re 29 316 offenbart. In US-4 100 140, US-4 127 615, US-4 130 600 und US-4 131 632 werden z. B. vernetzte Polymere erhalten durch Umsetzung von Polymaleinimiden mit Alkenylphenolen oder Alkenylphenolethern, gegebenenfalls in Anwesenheit von Epoxidharzen, beschrieben. Das bevorzugte Bis-maleinimid ist in vielen dieser Systeme N,N'-4,4'-diphenylmethan-bis-maleinimid. Solche Bis-maleinimide und aus ihnen hergestellte Präpolymere sind allerdings in üblichen organischen Lösungsmitteln schlecht löslich und die erhaltenen Lösungen sind nicht stabil. Eine gute Löslichkeit und Stabilität der Systeme sind aber sehr erwünscht, um verbesserte Verarbeitungsbedingungen zu erreichen.

Es wurde nun gefunden, dass die Verwendung von Diaminophenylindan-bis-maleinimid Produkte mit wesentlich besseren Eigenschaften erhalten werden. Sowohl dieses Bis-imid als auch die resultierenden Gemische und Präpolymere sind in üblichen organischen Lösungsmitteln, wie z. B. Methylethylketon, Aceton, Methylenchlorid usw., gut löslich. Die erhaltenen Stoffe sind sowohl in der Schmelze, als auch in der Lösung bei Raumtemperatur wie auch bei erhöhter Temperatur längere Zeit stabil. Die Präpolymere weisen eine überraschend hohe Reaktivität mit Aminen auf und können daher bei tieferen Temperaturen (z. B. bei 177 - 200°C) gehärtet werden. Dabei werden hochtemperaturresistente vernetzte Polymere mit guten mechanischen, thermischen und elektrischen Eigenschaften erhalten. So haben die vernetzten Polymere z. B. Glasumwandlungstemperaturen von über 300°C. Dieselben Produkte werden durch Härtung der Gemische erhalten. Diese Systeme sind daher für die Herstellung von hochleistungsfähigen Verbundstoffen und für ähnliche Anwendungen besonders gut geeignet.

Gegenstand der Erfindung sind härtbare Zusammensetzungen enthaltend ein Gemisch oder ein Präpolymer-Reaktionsprodukt von (a) Diaminophenylindan-bis-maleinimid und (b) einem Amin, einem Alkenylphenol, einem Alkenylphenolether oder deren Gemischen.

Das Diaminophenylindan-bis-maleinimid entspricht der Formel

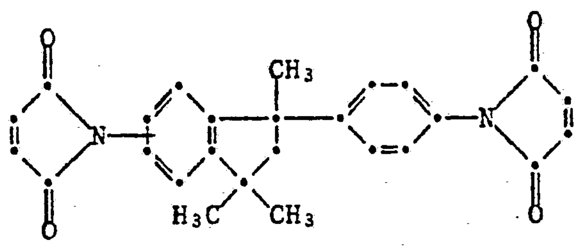

und kann durch Umsetzung von Phenylindandiamin und Maleinsäureanhydrid in Anwesenheit eines tertiären Amins, wie z. B. Triethylamin, und eines Lösungsmittels, wie z. B. Aceton, hergestellt werden. Eine weitere Herstellungsmethode ist in US-4 130 564 beschrieben.

Für die erfindungsgemässen Zusammensetzungen geeignete Amine sind z. B. aliphatische, cycloaliphatische oder aromatische primäre und sekundäre Amine, wobei aromatische Amine, insbesondere $C_6$-$C_{10}$-Arylendiamine, bevorzugt werden. Typische Amine sind z. B. Monoethanolamin, Ethylendiamin, Hexamethylendiamin, Trimethylhexamethylendiamin, Diethylentriamin, Triethylentetramin, Tetraethylenpentamin, N,N-Dimethylpropylendiamin-1,3, N,N-Diethylpropylendiamin-1,3, Bis(4-amino-3-methylcyclohexyl)methan, Bis(4-aminocyclohexyl)-methan, 2,2-Bis(4-aminocyclohexyl)propan, 3,5,5-Trimethyl-s-(aminomethyl)cyclohexylamin, N-Aminoethylpiperazin, m-Phenylendiamin, p-Phenylendiamin, Bis(4-aminophenyl)methan, Bis(4-aminophenyl)sulfon, m-Xylylendiamin, 1,2-Diaminocyclohexan, 1,4-Diaminocyclohexan, 1,3-Bis(aminomethyl)cyclohexan, 1,4-Bis(aminomethyl)cyclohexan, Isophorondiamin (3-Aminomethyl-3,5,5-trimethylcyclohexylamin), 1-Methylimidazol und Diaminophenylindan. Auch Polyamide können verwendet werden und sie fallen unter die erfindungsgemässe Definition der "Amine".

Bevorzugte aromatische Diamine sind z. B. $C_6$-$C_{10}$-Arylendiamine, wie z. B. p-Phenylendiamin, m-Phenylendiamin, m-Xylylendiamin und insbesondere Bis(4-Aminophenyl)methan und Diaminophenylindan.

Bevorzugte Alkenylphenole oder Alkenylphenolether sind z. B. Allylphenole, Methallylphenole und Propenylphenole. Es können einkernige und mehrkernige, insbesondere zweikernige Alkenylphenole und Alkenylphenolether verwendert werden, wobei vorzugsweise mindestens ein Kern sowohl eine Alkenylgruppe als auch eine phenolische, gegebenenfalls veretherte OH Gruppe enthält.

Es ist bekannt, dass Alkenylphenole durch thermische Umlagerung von Alkenylethern von Phenolen (z. B. des Allylphenylethers), hergestellt werden können (Claisenumlagerung). Diese Alkenylether können auch auf bekannte Weise durch Umsetzung von Phenolen und z. B. Allylchlorid in Anwesenheit eines Alkalimetallhydroxids in einem Lösungsmittel hergestellt werden.

Bevorzugte Alkenylphenole sind Verbindungen der Formeln I, II oder III:

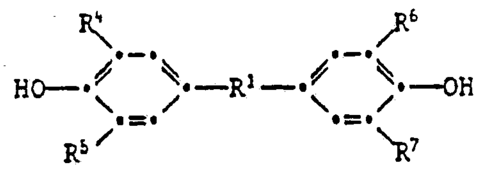

(I),

worin
$R^1$ eine direkte Bindung, Methylen, Isopropyliden, -O-, -S-, -SO-, oder $-SO_2-$ und
$R^4$, $R^5$, $R^6$ und $R^7$ unabhängig voneinander Wasserstoff oder $C_2$-$C_{10}$-Alkenyl, vorzugswseise Allyl oder Propenyl, sind, wobei mindestens einer der Reste $R^4$ bis $R^7$ eine Alkenylgruppe ist;

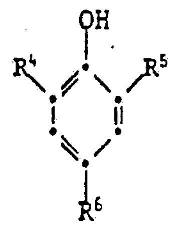

(II),

worin
$R^4$, $R^5$ und $R^6$ unabhängig voneinander Wasserstoff oder $C_2$-$C_{10}$-Alkenyl, vorzugsweise Allyl oder Propenyl, sind, wobei mindestens einer der Reste $R^4$ bis $R^6$ Alkenyl ist; und

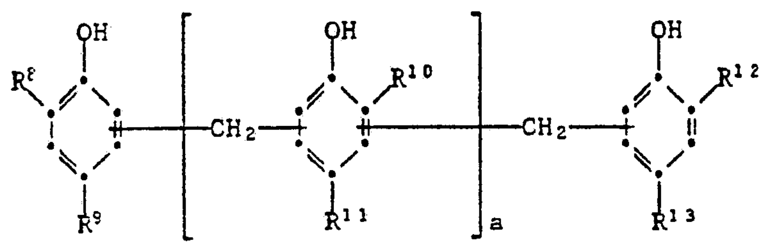

(III),

worin
$R^8$, $R^9$, $R^{10}$, $R^{11}$, $R^{12}$ und $R^{13}$ unabhängig voneinander Wasserstoff, $C_1$-$C_4$-Alkyl oder $C_2$-$C_{10}$-Alkenyl, vorzugsweise Allyl oder Propenyl, sind, wobei mindestens einer der Reste $R^8$ bis $R^{13}$ Alkenyl ist, und
a eine Zahl von 0 bis 10 ist;
oder Ether der Verbindungen der Formel I bis III, welche mindestens einen $-OR^3$-Rest enthalten, wobei $R^3$ $C_1$-$C_{10}$-Alkyl, $C_6$-$C_{10}$-Aryl oder $C_2$-$C_{10}$-Alkenyl ist.

Von den Verbindungen der Formel I werden solche bevorzugt, worin die Reste $R^4$ und $R^6$ je eine Propenylgruppe sind, die Reste $R^5$ und $R^7$ je ein Wasserstoffatom darstellen und $R^1$ Methylen, Isopropyliden oder -O- ist.

Alkenylsubstituierte Phenole und Polyole werden in den US-4 100 140 und 4 371 719 beschrieben. Typische Verbindungen umfassen z. B. o,o'-Diallylbisphenol A, 4,4'-Dihydroxy-3,3'-diallylbiphenyl, Bis-(4-hydroxy-3-allylphenyll)methan, 2,2-Bis(4-hydroxy-3,5-diallylphenyl)propan, Eugenol (4-Allyl-2-methoxyphenol), o,o'-Dimethallylbisphenol A, 4,4'-Dihydroxy-3,3'-dimethallylbiphenyl, Bis-(4-hydroxy-3-methallylphenyl)methan, 2,2-Bis(4-hydroxy-3,5-dimethallylphenyl)propan, 4-Methallyl-2-methoxyphenol, 2,2-Bis(4-methoxy-3-allylphenyl)-propan, 2,2-Bis(4-methoxy-3-methallylphenyl)propan, 4,4'-Dimethoxy-3,3'-diallylbiphenyl, 4,4'-Dimethoxy-3,3'-dimethallylbiphenyl, Bis(4-methoxy-3-allylphenyl)methan, Bis(4-methoxy-3-methallylphenyl)methan, 2,2-Bis(4-methoxy-3,5-diallylphenyl)propan, 2,2-Bis(4-methoxy-3,5-dimethallylphenyl)propan, 4-Allylveratrol (4-Allyl-1,2-dimethoxybenzol) und 4-Methallylveratrol (4-Methallyl-1,2-dimethoxybenzol). Besonders bevorzugt ist o,o'-Diallylbisphenol A.

Es können erfindungsgemäss auch Isomerengemische von propenyl- und allylsubstituierten ein- und mehrwertigen Phenolen eingesetzt werden. Bevorzugt werden Gemische von Propenyl- und allylsubstituierten Phenolen der Formel I eingesetzt, insbesondere solche der Formel Ia

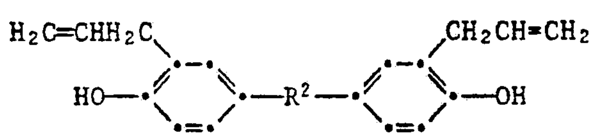

(Ia),

worin

$R^2$ Methylen, Isopropyliden oder 0 ist.

Gute Resultate werden auch mit Gemischen von mehrkernigen Alkenylphenolen und/oder Alkenylphenolethern mit einkernigen Alkenylphenolen und/oder Alkenylphenolethern erreicht.

Es können auch Gemische von Verbindungen, welche nur eine OH-Gruppe und nur eine Alkenylgruppe pro aromatischen Kern enthalten, mit Verbindungen, welche mehrere OH-Gruppen und/oder mehrere Alkenylgruppen pro aromatischen Kern aufweisen, oder Gemische der entsprechenden Phenylether dieser Verbindungen verwendet werden. Auch die entsprechenden Methallylverbindungen können eingesetzt werden.

Vorzugsweise werden solche Mengen der Komponente (b) eingesetzt, dass pro 1 Mol des Maleinimids 0,05 bis 2,0 Mol, vorzugsweise 0,1 bis 1,0 Mol und besonders bevorzugt 1 Mol des Amins oder des Alkenylphenols, des Alkenylphenolethers oder deren Gemische eingesetzt werden.

Erfindungsgemäss können als Komponente (b) Gemische von Aminen und Alkenylphenolyn bzw. Alkenylphenolethern verwendet werden. Dabei werden Gemische bevorzugt, worin die Komponente (b) ein 19 : 1 bis 1: 19, insbesondere ein 9 : 1 bis 1 : 9 Gemisch des Amins mit dem Phenol ist, wobei es sich bei den Teilen um Gewichtsteile handelt.

Besonders bevorzugt sind Gemische von o-o'-Diallylbisphenol A mit Bis-(4-aminophenyl)methan oder mit Diaminophenylindan.

Die erfindungsgemässen Zusammensetzungen können durch blosses Zusammenmischen der Komponenten, oder durch Erhitzen des Gemisches bei 75 bis 130°C während etwa 15 bis 60 Minuten, um das Präpolymer-Reaktionsprodukt zu erzeugen, hergestellt werden. Um die Umsetzung zu erleichtern, können auch Lösungsmittel, besonders flüchtige Lösungsmittel, wie chlorierte Kohlenwasserstoffe, Ester, Etheralkohole oder Tetrahydrofuran eingesetzt werden. Das Lösungsmittel wird nach der Umsetzung entfernt, und das gewünschte Präpolymer-Reaktionsprodukt erhalten.

Die Härtung der erfindungsgemässen Zusammensetzungen ist an sich bekannt. Sie erfolgt geeigneterweise bei Temperaturen zwischen 100 und 250°C während einer für die Härtung ausreichenden Zeit.

Während der Härtung entsteht ein Netzwerk mit hoher Vernetzungsdichte. Der hier verwendete Ausdruck Härtung bedeutet daher die Umwandlung von Gemischen oder Präpolymeren in unlösliche und nichtschmelzbare vernetzte Produkte, mit gleichzeitiger Formung zu geformten Artikeln wie z. B. Giesskörpern, Presskörpern, Laminaten, oder zu zweidimensionalen Strukturen, wie z. B. Überzügen, Emaillen und Klebverbindungen. Die hergestellten Überzüge zeichnen sich z. B. durch erhöhte Zähigkeit aus.

Die erfindungsgemässen Zusammensetzungen können in jeder Verarbeitungsphase vor der Härtung mit den üblichen Modifizierungsmitteln vermischt werden, wie z. B. Streckmittel, Füllstoffe und Verstärkungsmittel, Pigmente, Farbstoffe, organische Lösungsmittel, Plastifizierungsmittel, Mittel zur Verbesserung der Trockenklebrigkeit (Klebrigmacher), Gummis, Beschleuniger, Verdünner usw. Als Streckmittel, Verstärkungsmittel, Füllstoffe und Pigmente kommen z. B. in Frage: Kohleteer, Bitumen, Glasfasern, Borfasern, Kohlefasern, Cellulose, Polyethylenpulver, Polypropylenpulver, Glimmer, Asbest, Quarzpulver, Gips, Antimontrioxid, Bentone, Siliziumdioxidaerogel ("Aerosil"), Lithopon, Barit, Titandioxid, Russ, Graphit, Eisenoxid oder Metallpulver wie z. B. Aluminiumpulver oder Eisenpulver. Den härtbaren Zusammensetzungen können auch andere übliche Zusätze, wie z. B. Flammschutzmittel, Thixotropiemittel, Verlaufsmittel, wie Silicone, Celluloseacetatbutyrat, Polyvinylbutyrat, Wachse, Stearate und ähnliches (welche zum Teil auch als Formtrennmittel verwendet werden) beigegeben werden.

Wenn die erfindungsgemässen Zusammensetzungen z. B. als Klebstoffformulierungen verwendet werden, können auch carboxylgruppenendständiger Acrylnitril-Butadien-Kautschuk, Modifizierungsharze, wie Triglycidyl-p-aminophenol und Beschleuniger wie Bortrifluorid-Monoethylaminkomplexen oder Imidazolkomplexe beigegeben werden.

Die härtbaren Zusammensetzungen können auf übliche Art unter Verwendung bekannter Mischaggregate, wie Rührer, Kneter, Walzkörper und ähnliches, hergestellt werden.

Die erfindungsgemässen Zusammensetzungen zeichnen sich durch hohe Reaktivität, gute Löslichkeit in üblichen Lösungsmitteln, gute Stabilität in der Schmelze oder in Lösung sowie durch gute thermische und mechanische Eigenschaften der gehärteten Produkte, z. B. gute Biegen und Scherfestigkeit oder interlaminare Scherfestigkeit aus. Die erhaltenen Produkte weisen gute mechanische, thermische und elektrische Eigenschaften auf, haben hohe Glasumwandlungstemperaturen und sind im wesentlichen nicht brüchig. Die erfindungsgemässen Zusammensetzungen können auch problemlos als Schmelze, besonders ohne Zugabe von nichtflüchtigen Lösungsmitteln, z. B. für die Imprägnierung, verwendet werden.

Gegenstand der vorliegenden Erfindung sind daher auch Produkte, erhältlich durch Härtung der erfindungsgemässen Zusammensetzungen, sowie Laminate, erhältlich durch Erhitzen eines mit der erfindungsgemässen Zusammensetzung imprägnierten Materials.

Die beschriebenen erfindungsgemässen Zusammensetzungen können auf verschiedenen Gebieten eingesetzt werden, wie z. B. in Verbundstoffen, Leiterplatten, Giesslingen, Formkörpern, Klebstoffen und Überzügen.

Von besonderem Interesse ist ihre Anwendung für die Herstellung von Hochleistungsverbundstoffen, welche z. B. in der Luftfahrtindustrie sehr wichtig sind. So können die modifizierten Harze zum Präimprägnieren von verschiedenem faserartigem Material verwendet werden, welches als Deckschicht für Honigwabenstrukturen oder als Strukturteile eingesetzt werden. Verfahren zur Herstellung von Prepregs sind dem Fachmann bekannt. Als faserartige Materialien können z. B. Graphit, Glas, Kevlar® verwendet werden. Auch Verfahren zur Herstellung von Laminaten sind bekannt. Laminate verschiedenster Dicken können z. B. durch Pressformen oder Autoklavformen hergestellt werden. Die erfindungsgemässen Zusammensetzungen können auch erfolgreich als hatfvermittelnde Substanzen eingesetzt werden.

In den folgenden Beispielen sind einige bevorzugte Ausführungsformen der vorliegenden Erfindung beschrieben.

**Beispiel 1:**

Ein Präpolymer wird durch Umsetzung von 438 g (1 Mol) Diaminophenylindan-bis-maleinimid mit 308 g (1 Mol) o,o'-Diallylbisphenol A bei 100 - 120°C während 30 - 60 Minuten im Vacuum und mit ständigem Rühren hergestellt. Das Produkt ist eine klare viskose Flüssigkeit. Eine 50 gew.-%-ige Lösung des Präpolymers in Methylethylketon wird hergestellt, ohne dass eine Veränderung des Aussehens bemerkt wird. Nach 26 Wochen bei Raumtemperatur wird kein Absetzen von Feststoffen oder Veränderung der Viskosität bemerkt.

**Beispiel 2:**

Mit dem Präpolymer aus Beispiel 1 werden gehärtete Harzplatten hergestellt, indem zuerst während 15 Minuten im Vakuum entgast wird und die Schmelze in 8,5 mm dicke Formen gegossen und wie folgt gehärtert wird: 1 Stunde bei 180°C, 2 Stunden bei 200°C und 6 Stunden bei 250°C. Die erhaltene Platte ist vollständig ausgehärtet.

**Beispiel 3:**

Die Glasumwandlungstemperatur des nach Beispiel 2 ausgehärteten Harzes wird auf einem Perkin-Elmer TMA Instrument bestimmt (20°C/Min., Eindringen eines 40 g Gewichtes) und beträgt 297°C. Bei Raumtemperatur werden auch die Zug- und Biegeeigenschaften des Harzes nach ASTM D-638 bzw. ASTM D-790 gemessen:

| | |
|---|---|
| Zugfestigkeit ($N/mm^2$) | 62,0 |
| Zugmodul ($N/mm^2$) | 3955 |
| Zugdehnung (%) | 1.8 |
| Biegefestigkeit ($N/mm^2$) | 119,9 |
| Biegemodul ($N/mm^2$) | 3865 |

Aus den erwähnten Daten sind die vorzüglichen Eigenschaften der erfindungsgemässen Zusammensetzungen ersichtlich.

**Beispiel 4:**

Das im Beispiel 1 beschriebene Vorgehen wird wiederholt, mit der Ausnahme, dass dem Gemisch noch 37,8 g Bis(4-aminophenyl)methan beigegeben werden. Das erhaltene Präpolymer ist bei 75 - 130°C eine klare viskose Flüssigkeit.

**Beispiel 5:**

Das im Beispiel 4 beschriebene Vorgehen wird wiederholt, mit der Ausnahme, dass statt Bis(4-aminophenyl)methan Diaminophenylindan verwendet wird. Das erhaltene Präpolymer ist bei 75 - 130°C eine klare, viskose Flüssigkeit.

**Beispiel 6:**

Die Gelierzeit, welche die Reaktivität des Systems zeigt, wird bestimmt. Jedes System wird in einen offenen Behälter gestellt und auf einer Heizplatte bei 177°C erhitzt. Die für die Gelbildung benötigte Zeit wird vermerkt. Kürzere Zeiten bedeuten die Möglichkeit einer schnelleren Verarbeitung, keinen Bedarf an Härtungskatalysatoren und eine geringere Wahrscheinlichkeit des Ausblutens oder anderer unerwünschter Eigenschaften.

Beispiel 1 Gelierzeit 14,5 Min.
Beispiel 4 Gelierzeit 3,0 Min.
Beispiel 5 Gelierzeit 4,0 Min.

**Beispiel 7:**

Ein Präpolymer wird aus 1 Mol Diaminophenylindan-bis-maleinimid und 1 Mol Bis(4-aminophenyl)methan hergestellt und mit einem Du-Pont Differential Scanning Kalorimeter auf Reaktivität und Stabilität der Schmelze untersucht. Der Test wird unter Stickstoff mit einer Aufheizgeschwindigkeit von 10°C/Min. durchgeführt, und dabei wird ein Maximum bei 115°C gemessen. Die tiefe beobachtete Temperatur des Systems ist für die gewünschte schnelle Härtung, einfache Verarbeitung und grosse Stabilität charakteristisch.

Zudem zeigen die Endotherm-Exotherm-Kurven eine schnelle, steile Neigung vom Punkt des Maximalen Endotherm zum Punkt des maximalen Exotherm. Dieses Verhalten ist für eine ausgezeichnete Stabilität der Schmelze charakteristisch, da sich die Schmelze schnell verfestigt und sich daher nicht zersetzen kann.

Zusammenfassend kann gesagt werden, dass die erfindungsgemässen Maleinimidsysteme im Vergleich mit anderen Maleinimidsystgemen verbesserte Eigenschaften aufweisen.

**Patentansprüche**

1. Härtbare Zusammensetzung enthaltend ein Gemisch oder ein Präpolymer-Reaktionsprodukt von (a) Diaminophenylindan-bis-maleinimid und (b) einem Amin, einem Alkenylphenol, einem Alkenylphenolether oder deren Gemischen.

2. Zusammensetzung nach Anspruch 1, worin pro Mol der Komponente (a) 0,05 bis 2,0, vorzugsweise ein Mol der Komponente (b) enthalten sind.

3. Zusammensetzng nach Anspruch 1, worin die Komponente (b) ein $C_6$-$C_{10}$-Arylendiamin und insbesondere Bis(4-aminophenyl)methan oder Diaminophenylindan ist.

4. Zusammensetzung nach Anspruch 1, worin die Komponente (b) ein Alkenylphenol der Formeln I, II oder III ist:

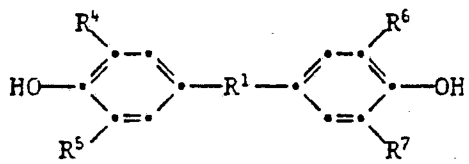

(I),

worin

$R^1$ eine direkte Bindung, Methylen, Isopropyliden, -O-, -S-, -SO- oder -$SO_2$- und

$R^4$, $R^5$, $R^6$ und $R^7$ unabhängig voneinander Wasserstoff oder $C_2$-$C_{10}$-Alkenyl sind, wobei mindestens einer der Reste $R^4$ bis $R^7$ eine Alkenylgruppe ist;

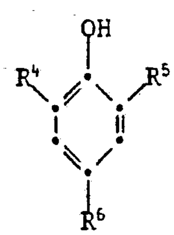

(II),

worin

$R^4$, $R^5$ und $R^6$ unabhängig voneinander Wasserstoff oder $C_2$-$C_{10}$-Alkenyl sind, wobei mindestens einer der Reste $R^4$ bis $R^6$ Alkenyl ist; und

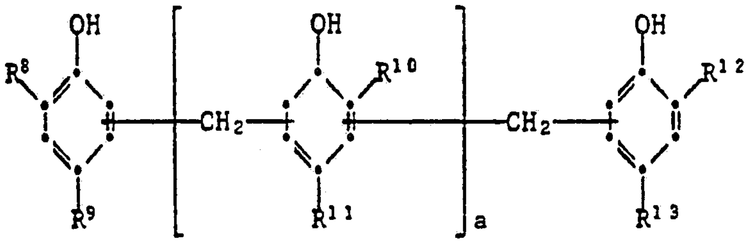

(III),

worin

$R^8$, $R^9$, $R^{10}$, $R^{11}$, $R^{12}$, und $R^{13}$ unabhängig voneinander Wasserstoff, $C_1$-$C_4$-Alkyl oder $C_2$-$C_{10}$-Alkenyl sind, wobei mindestens einer der Reste $R^8$ bis $R^{13}$ Alkenyl ist, und

a eine Zahl von 0 bis 10 ist;

oder Ether der Verbindungen der Formel I bis III, welche mindestens einen $-OR^3$-Rest enthalten, wobei $R^3$ $C_1$-$C_{10}$-Alkyl, $C_6$-$C_{10}$-Aryl oder $C_2$-$C_{10}$-Alkenyl ist.

5. Zusammensetzung nach Anspruch 4, worin die Alkenylgruppe Allyl, Methallyl oder Propenyl ist.

6. Zusammensetzung nach Anspruch 5, worin das Alkenylphenol o,o'-Diallylbisphenol A ist.

7. Zusammensetzung nach Anspruch 1, worin die Komponente (b) ein 19 : 1 bis 1 : 19, insbesondere ein 9 : 1 bis 1 : 9 Gemisch des Amins mit dem Phenol ist, wobei es sich bei den Teilen um Gewichtsteile handelt.

8. Zusammensetzung nach Anspruch 7, worin die Komponente (b) ein Gemisch von o,o'-Diallylbisphenol A mit Bis(4-aminophenyl)methan oder mit Diaminophenylindan ist.

9. Produkt erhältlich durch Härtung der Zusammensetzung nach Anspruch 1.

10. Laminat erhältlich durch Erhitzen eines mit der Zusammensetzung nach Anspruch 1 imprägnierten Materials.

## Claims

1. A curable composition containing a mixture or prepolymer reaction product of (a) diaminophenylindane-bis-maleinimide, and (b) an amine, an alkenyl phenol, an alkenyl phenol ether or mixtures thereof.

2. The composition according to claim 1, wherein 0.05 to 2.0 moles, preferably one mole of component (b) are present per mole of component (a).

3. The composition according to claim 1, wherein component (b) is a $C_6$-$C_{10}$arylene diamine and is in particular bis(4-aminophenyl)methane or diaminophenylindane.

4. The composition according to claim 1, wherein component (b) is an alkenyl phenol of the formulae I, II or III:

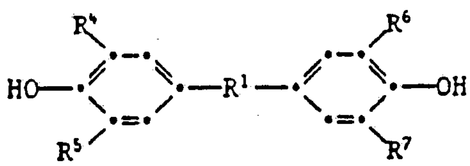

(I),

wherein

$R^1$ is a direct bond, methylene, isopropylidene, -O-, -S-, -SO- or $-SO_2-$, and

$R^4$, $R^5$, $R^6$ and $R^7$ are independently of one another hydrogen or $C_2$-$C_{10}$alkenyl, at least one of the radicals $R^4$ to $R^7$ being an alkenyl group;

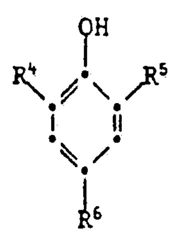

(II),

wherein

$R^4$, $R^5$ and $R^6$ are independently of one another hydrogen or $C_2$-$C_{10}$alkenyl, at least one of the radicals $R^4$ to $R^6$ being alkenyl; and

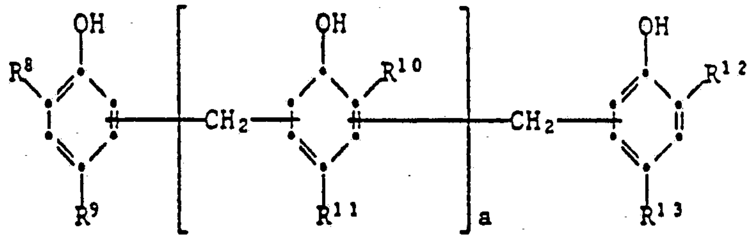

(III),

wherein

$R^8$, $R^9$, $R^{10}$, $R^{11}$, $R^{12}$ and $R^{13}$ are independently of one another hydrogen, $C_1$-$C_4$alkyl or $C_2$-$C_{10}$alkenyl, at least one of the radicals $R^8$ to $R^{13}$ being alkenyl, and

a is a number from 0 to 10;

or ethers of the compounds of the formula I to III containing at least one -$OR_3$ radical, $R_3$ being $C_1$-$C_{10}$alkyl, $C_6$-$C_{10}$aryl or $C_2$-$C_{10}$alkenyl.

5. The composition according to claim 4, wherein said alkenyl group is allyl, methallyl or propenyl.

6. The composition according to claim 5, wherein said alkenyl phenol is o,o'-diallylbisphenol A.

7. The composition according to claim 1, wherein component (b) is a 19 : 1 to 1 : 19, in particular 9 : 1 to 1 : 9, mixture of the amine and the phenol, parts being parts by weight.

8. The composition according to claim 7, wherein component (b) is a mixture of o,o'-diallylbisphenol A with bis(4 aminophenyl)methane or with diaminophenylindane.

9. The product obtainable by curing the composition according to claim 1.

10. A laminate obtainable by heating a material impregnated with the composition according to claim 1.

**Revendications**

1. Composition durcissable contenant un mélange ou un produit réactionnel prépolymère constitué (a) d'un bis-maléimido-phénylindane et (b) d'une amine, d'un alcénylphénol, d'un éther d'alcénylphénol ou de leurs mélanges.

2. Composition selon la revendication 1 dans laquelle il y a, par mole de la composante (a), de 0,05 à 2,0 mol, de préférence 1 mol, de la composante (b).

3. Composition selon la revendication 1 dans laquelle la composante (b) est une arylène-diamine en $C_6$-$C_{10}$, en particulier le bis-(amino-4 phényl)-méthane ou un diaminophénylindane.

4. Composition selon la revendication 1 dans laquelle la composante (b) est un alcénylphénol répondant à l'une des formules I, II et III:

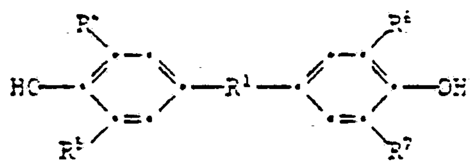

(I)

formule dans laquelle

$R^1$ représente une liaison directe, un méthylène, un isopropylidone, -O-, -S-, -SO- ou -$SO_2$, et

$R^4$, $R^5$, $R^6$ et $R^7$ représentent chacun, indépendamment les uns des autres, l'hydrogène ou un alcényle en $C_2$-$C_{10}$, au moins un des symboles $R^4$ à $R^7$ représentant un radical alcényle;

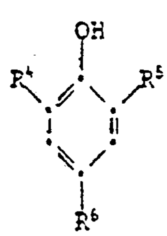

(II)

dans laquelle

$R^4$, $R^5$ et $R^6$ représentent chacun, indépendamment les uns des autres, l'hydrogène ou un alcényle en $C_2$-$C_{10}$, au moins un des symboles $R^4$ à $R^6$ représentant un alcényle, et

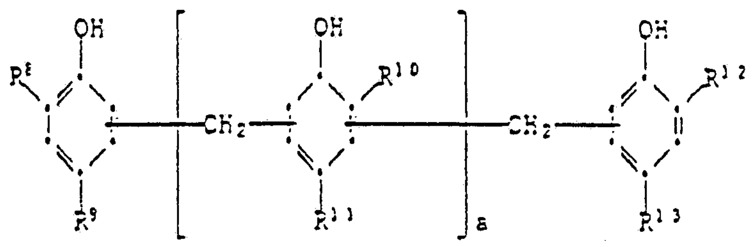

(III)

dans laquelle

$R^8$, $R^9$, $R^{10}$, $R^{11}$, $R^{12}$ et $R^{13}$ représentent chacun, indépendamment les uns des autres, l'hydrogène, un alkyle en $C_1$-$C_4$ ou un alcényle en $C_2$-$C_{10}$, au moins un des symboles $R^8$ à $R^{13}$ représentant un radical alcényle, et

a représente un nombre de 0 à 10

ou des éthers des composés de formules I à III qui contiennent au moins un radical $-OR^3$ dans lequel $R^3$ désigne un alkyle en $C_1$-$C_{10}$, un aryle en $C_6$-$C_{10}$ ou un alcényle en $C_2$-$C_{10}$.

5. Composition selon la revendication 4 dans laquelle le radical alcényle est un radical allyle, méthallyle ou propényle.

6. Composition selon la revendication 5 dans laquelle l'alcénylphénol est l'o,o'-diallyl-bis-phénol A.

7. Composition selon la revendication 1 dans laquelle la composante (b) est un mélange de l'amine et du phénol dans un rapport pondéral compris entre 19 : 1 et 1 : 19, plus particulièrement entre 9 : 1 et 1 : 9.

8. Composition selon la revendication 7 dans laquelle la composante (b) est un mélange d'o,o'-diallyl-bis-phénol A et de bis-(amino-4 phényl)-méthane ou d'un diaminophénylindane.

9. Produit qui peut être obtenu par durcissement de la composition selon la revendication 1.

10. Stratifié qui peut être obtenu par chauffage d'une matière qui a été imprégnée avec la composition selon la revendication 1.